# EUROPÄISCHE PATENTSCHRIFT

(11) **EP 1 381 850 B1**
(45) Veröffentlichungstag und Bekanntmachung des Hinweises auf die Patenterteilung: **23.12.2009**
(21) Anmeldenummer: 02745253.1
(22) Anmeldetag: 22.04.2002
(51) Int. Cl.: G01N 21/85, G01N 33/02

(54) **VERFAHREN UND VORRICHTUNG ZUR GETREIDEANALYSE**
METHOD AND DEVICE FOR ANALYSING CEREALS
PROCEDE ET DISPOSITIF D'ANALYSE DE CEREALES

(30) Priorität: 23.04.2001 DE 10119763
(43) Veröffentlichungstag der Anmeldung: 21.01.2004
(73) Patentinhaber: Bruins, Hans Joachim, 81247 München (DE)
(72) Erfinder: Bruins, Hans Joachim, 81247 München (DE)
(74) Vertreter: Hertz, Oliver
(86) Internationale Anmeldenummer: PCT/EP2002/004424
(87) Internationale Veröffentlichungsnummer: WO 2002/086473

(56) Entgegenhaltungen:
- EP-A- 0 388 082
- WO-A-98/45678
- JP-A- 8 338 809
- JP-A- 08 285 763
- US-A- 3 328 587
- US-A- 4 479 055
- US-A- 4 563 581
- US-A- 5 537 202
- US-A- 5 591 461

## Beschreibung

Die Erfindung betrifft ein Verfahren zur Getreideanalyse, insbesondere zur Messung spektroskopischer Eigenschaften von Getreidekörnern, und eine Fördereinrichtung, insbesondere für Getreidekörner, mit einer Messeinrichtung zur Umsetzung des Messverfahrens.

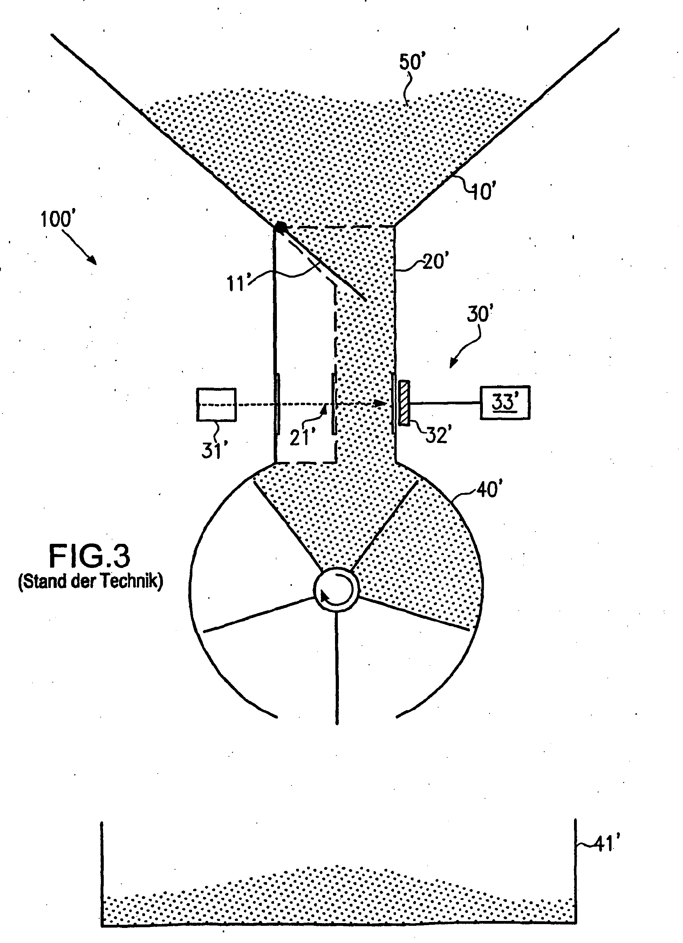

Es ist üblich, beispielsweise unmittelbar nach der Ernte oder bei anschließenden Gelegenheiten bei der Lagerung oder dem Transport Getreide einer Analyse zu unterziehen, die auf die Erfassung z. B. des Feucht- und Proteingehalts der Getreidekörner gerichtet ist. Aus der Praxis ist bekannt, hierzu eine spektroskopische Messung am Getreide durchzuführen, die im folgenden unter Bezug auf Fig. 3 erläutert wird. Eine herkömmliche Getreidefördereinrichtung 100' umfasst eine Zufuhreinrichtung mit einem Trichter 10', einen Fallschacht 20' mit einer Messeinrichtung 30', die eine Lichtquelle 31', einen Sensor 32' und eine Auswertungseinheit 33' aufweist, und eine Abfuhreinrichtung mit einem Schaufelrad 40'. Die Wand des Fallschachts 20' ist im Bereich einer Messstrecke 21' für das Messlicht der Lichtquelle 31' durchlässig.

Zur Getreideanalyse wird Getreide 50' vom Trichter 10' über eine Klappe 11' in den Fallschacht 20' geleitet. Der Fallschacht 20' ist so bemessen, dass das zugeführte Getreide durch den Fallschacht 20' hindurch rutscht, wobei es im Bereich der Messstrecke 21' einer spektroskopischen Messung unterzogen wird. Nach Durchtritt durch den Fallschacht 20' wird das Getreide mit dem Schaufelrad 40' in eine Schublade 41' weiterbefördert. Die spektroskopische Messung umfasst beispielsweise die Messung der optischen Dichte der Getreideschicht im Fallschacht 20'.

Die in Fig. 3 illustrierte Technik besitzt die folgenden Nachteile. Das Hauptproblem besteht darin, dass die Fülldichte des Getreides im Fallschacht 20' insbesondere im Bereich der Messstrecke 21' nicht konstant ist. Beispielsweise durch Feuchtigkeit im Getreide kommt es dazu, dass Getreide an der Klappe 11' verklebt und nicht in den Fallschacht 20' fällt. Des weiteren kann es im Fallschacht 20' zu Verstopfungen kommen. Insbesondere bei der Messung an Gerste, deren Körner sich durch relativ lange Spelzen auszeichnen, besteht die Gefahr, dass das Getreide nicht zum Schaufelrad 40' weiterrutscht und stattdessen im Schaufelrad 20' ein Gewölbe bildet. Durch die Messung am bewegten Getreide ist also eine gegebenenfalls von Messung zu Messung veränderte Fülldichte gegeben, wodurch die Genauigkeit und Reproduzierbarkeit der spektroskopischen Messung eingeschränkt wird.

Ein weiterer Nachteil der herkömmlichen Technik betrifft die Messwertverarbeitung an sich. Die optische Dichte des Getreides wird relativ zur optischen Dichte der leeren Messstrecke 21' erfasst. Beide Dichtewerte unterscheiden sich so stark, dass die Referenzmessung mit einem Abschwächer (nicht dargestellt) erfolgen oder mit einer geringen Verstärkung erfasst werden muss. Der Einsatz des Abschwächers besitzt den Nachteil einer erheblich verlängerten Messzeit. Wird hingegen für die Referenzmessung ein Verstärker in der Auswertungseinheit 33' umgeschaltet, so wird dabei auch eine Kennlinienänderung eingeführt, die bei der herkömmlichen Signalanalyse durch Quotientenbildung aus Mess- und Referenzsignal eine Fehlerquelle für die Messwertanalyse bildet.

Schließlich besteht ein Nachteil der in Figur 3 illustrierten Messtechnik darin, dass diese auf die Messung an Getreide beschränkt ist. Es kann nicht ohne Weiteres auf die Messung an Getreideprodukten (z. B. Mehl) umgeschaltet werden, da die Messung an Mehl eine geringere Schichtdicke bei der optischen Messung und einen anderen Transportvorgang erfordert. Es besteht jedoch häufig ein Interesse beim Anwender, mit einer flexiblen Messeinrichtung zu arbeiten, die sowohl für Getreide als auch für Getreideprodukte geeignet ist.

Aus US 4 479 055 ist eine Infrarotmesseinrichtung zur Analyse von riesel- oder fließfähigen Lebensmitteln bekannt, bei der das zu analysierende Material zur Messung in einer Bypassleitung eines Fallschachts aufgestaut wird. Eine unterhalb des Messfensters angeordnete Sperrvorrichtung ermöglicht die Entleerung des Bypasskanals und das Nachfließen von neuem Material. Alternativ wird zu messendes Material in eine Messküvette gefüllt, welche in einer an der Infrarotanordnung vorgesehene Aufnahme positioniert wird. Die Messküvette kann jedoch nicht in dem Fallschacht, beziehungsweise in dem Bypasskanal positioniert werden.

Die US 3 328 587 offenbart eine Vorrichtung zur optischen Analyse von pulverförmigen Materialien, welche mit Hilfe eines Schneckenantriebs in eine Messzelle befördert werden. Ein Druckventil zusammen mit der kontinuierlich laufenden Förderschnecke sorgt für eine sich kontinuierlich an einem Messfenster vorbei bewegende Materialsäule mit einer gleichbleibenden Verdichtung.

Aus der internationalen Anmeldung WO 98/45678 ist eine Vorrichtung und ein Verfahren zur optischen Bestimmung von Inhaltsstoffen eines rieselfähigen Gutes beschrieben. Der Transport des rieselfähigen Materials erfolgt in einem Fallschacht mit Hilfe der Schwerkraft. Eine Fördereinrichtung zum Transport des rieselfähigen Guts in den Fallschacht ist nicht offenbart.

Die US 5 537 202 offenbart eine Formungs- und Messvorrichtung für Pulverproben, bei welcher ein pulverförmiges Probenmaterial zunächst mit einem Schaufelrad transportiert wird, anschließend verschoben und durch Druck verdichtet wird, bevor sie einer optischen Messung unterzogen wird. Probentransport, Portionierung und Verdichtung werden in verschiedenen Schritten erzielt.

Die EP 0 388 082 A2 offenbart ein Infrarotspektrometer zur Untersuchung von Getreideproben, welche in einem mit einer Sperreinrichtung verschlossenen Fallschacht untersucht werden. Als Zufuhrvorrichtung ist ein Vibrationsschütter vorgesehen, womit die Probe nicht portioniert wird. Zur spektroskopischen Messung wird mit Hilfe eines optischen Filterrades Licht einer bestimmten Wellenlänge ausgewählt, und durch ein transparente Fenster in der Wand des Fallschachtes auf das Probenmaterial geleitet.

Aus der US 5 591 461 ist eine Vorrichtung zur Formung von Pulverproben bekannt, bei welcher das Probenmaterial durch eine Rohrleitung in einen mit einer Sperreinrichtung verschlossenen Fallschacht geleitet wird und anschließend mit einem Stempel verdichtet wird. Nach einer spektroskopischen Messung durch ein in dem Fallschacht angeordnetes Fenster wird die Sperreinrichtung geöffnet und die Probe mit Hilfe des Stempels aus dem Fallschacht bewegt.

Die US 4 563 581 offenbart einen Infrarotanalysator, bei welchem ein pulverförmiges Probenmaterial von einer Förderschnecke durch einen horizontal angeordneten Kanal transportiert wird. In dem Kanal befindet sich ein Fenster, auf dessen Außenseite eine spektroskopische Messeinrichtung angeordnet ist. Für die Messung wird der Probentransport durch die Förderschnecke jeweils gestoppt, so dass die Messung an einer ruhenden Probe erfolgen kann. Eine Portionierung der Probe ist nicht vorgesehen.

Aus der JP 08285763 ist ein Infrarotspektroskop für Pulverproben bekannt. Die Pulverprobe wird durch einen Trichter in einen vertikal angeordneten Fallschacht gefüllt und durch Druck von der Seite verdichtet. Die JP 08338809 offenbart eine Messvorrichtung zur Untersuchung von Getreideproben, bei welcher Getreidekörner in einem Fallschacht einer spektroskopischen Messung unterzogen werden, wobei das Getreide durch ein Ventil kornweise an eine Sortiereinheit weitertransportiert wird.

Die Aufgabe der Erfindung ist es ein verbessertes Verfahren zur Messung spektroskopischer Eigenschaften von Getreide anzugeben, mit dem die Nachteile der herkömmlichen Technik überwunden werden, das einen erweiterten Anwendungsbereich besitzt und das insbesondere Analysen ermöglicht, die sowohl schnell als auch mit hoher Genauigkeit und Reproduzierbarkeit durchgeführt werden. Die Aufgabe der Erfindung ist es auch, eine Getreidefördereinrichtung mit einer Messeinrichtung zur Umsetzung des Verfahrens anzugeben. Die Getreidefördereinrichtung soll insbesondere eine erhöhte Flexibilität in Bezug auf die Art der Probe besitzen.

Diese Aufgaben werden mit einem Verfahren und einer Vorrichtung mit Merkmalen gemäß den Patentansprüchen 1 und 8 gelöst. Vorteilhafte Ausführungsformen der Erfindung ergeben sich aus den abhängigen Ansprüchen.

Die Grundidee der Erfindung ist es insbesondere, abweichend von der herkömmlichen Messung am laufend bewegten Getreide ein Verfahren zur Getreideanalyse bereitzustellen, bei dem eine Messung spektroskopischer Eigenschaften an ruhendem Getreide erfolgt. Erfindungsgemäß wird eine Getreidefördereinrichtung derart betrieben, dass aufeinanderfolgend zunächst eine Probenmenge in einen Fallschacht mit einer Messstrecke eingefüllt wird, wobei der Fallschacht mit einer Sperreinrichtung verschlossen ist, so dass die Probe (Getreidekörner) mit einer vorbestimmten Fülldichte in der Messstrecke ruht, und in diesem Zustand die Messung von spektroskopischen Eigenschaften (z. B. Transmissionsmessung oder dgl.) erfolgt, woraufhin die Sperreinrichtung freigegeben und das gemessene Getreide in eine Abfuhreinrichtung befördert oder das gemessene Getreideprodukt aus der Messstrecke entnommen wird. Die Messung an ruhenden Proben besitzt den Vorteil, dass die Fülldichte bei jeder Messung gleich ist. Die Reproduzierbarkeit der. Messbedingungen und Messergebnisse wird damit erheblich erhöht.

Gemäß einer bevorzugten Ausführungsform der Erfindung erfolgt am ruhenden Getreide eine Messung der Transmission oder optischen Dichte. Es erfolgen die Messung der Probe und mindestens eine Referenzmessung, wobei die Messungen im Unterschied zur herkömmlichen Technik ohne Verwendung eines Abschwächers erfolgen. Erfindungsgemäß werden aus den Spektren S_{P} der Probe und S_{R} der Referenzmessung für jede Probenmessung ein Differenzspektrum ermittelt und dieses quantitativ ausgewertet.

Eine erfindungsgemäße Getreidefördereinrichtung mit einer Messeinrichtung zur Umsetzung des erfindungsgemäßen Verfahren zeichnet sich insbesondere durch einen Fallschacht aus, an dem die Messeinrichtung vorgesehen ist und der unterhalb der Messeinrichtung mit einer Sperreinrichtung zum zeitweiligen Verschließen des Fallschachtes ausgestattet ist. Es ist eine Steuereinrichtung vorgesehen, mit der abwechselnd die Messeinrichtung und eine Stelleinrichtung der Sperreinrichtung betätigt werden.

Erfindungsgemäß wird die Zufuhreinrichtung der Getreidefördereinrichtung durch ein Schaufelrad gebildet. Gemäß bevorzugten Ausführungsformen der Erfindung besteht die Wand des Fallschachts im Bereich der Messstrecke zumindest teilweise aus einem optischen Filtermaterial, das unempfindlich gegen elektrostatische Aufladungen ist, und/oder ist die Steuereinrichtung mit der Zufuhreinrichtung, insbesondere dem Schaufelrad, verbunden und dazu eingerichtet, in Abhängigkeit von vorbestimmten Messsignalen eine Schaufelradreinigung auszulösen.

Weitere Vorteile und Einzelheiten der Erfindung werden aus der Beschreibung der Zeichnungen ersichtlich. Es zeigen:
- Fig. 1:: eine schematische Darstellung der erfindungsgemäßen Getreidefördereinrichtung,
- Fig. 2:: eine Darstellung der Getreidefördereinrichtung mit eingesetzter Messzelle zur Analyse von Getreidepro- dukten (keine Ausführungsform der Erfindung), und
- Fig. 3:: eine schematische Darstellung einer herkömmlichen Getreidefördereinrichtung (Stand der Technik).

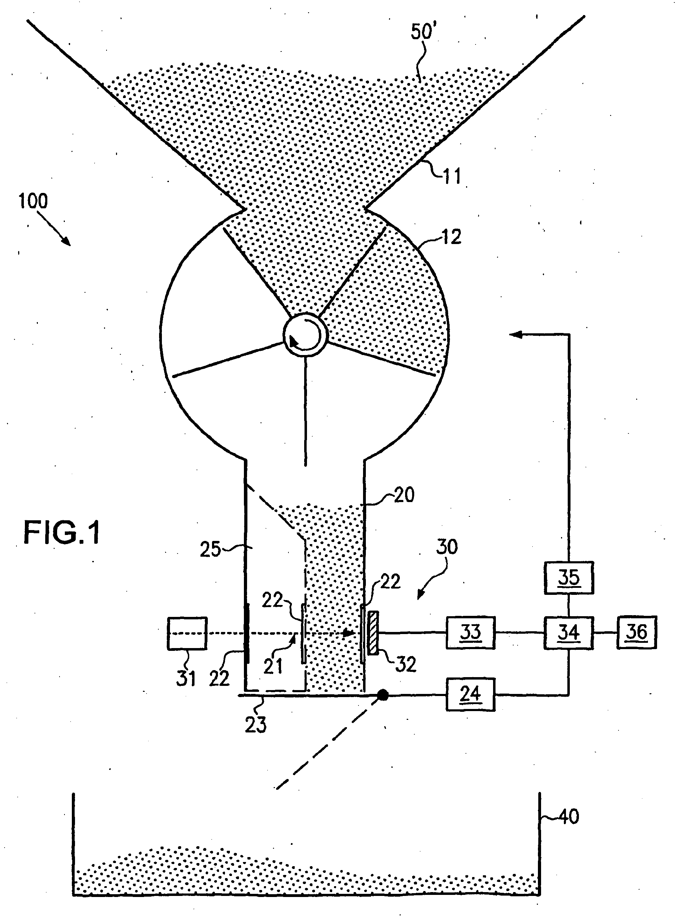

Die Getreidefördereinrichtung 100 gemäß Fig. 1 umfasst eine Zufuhreinrichtung mit einem Trichter 11 zur Aufnahme des Getreides 50 und einem Förderer 12. Der Förderer 12 wird durch das schematisch illustrierte Schaufelrad gebildet. Der Fallschacht 20 ist ein vertikal oder bauartabhängig auch geneigt angeordneter Schacht mit einem rechteckigen oder zumindest in Teilbereichen gegebenenfalls gekrümmten Querschnitt. Im Fallschacht 20 ist die Messstrecke 21 vorgesehen. Im Bereich der Messstrecke 21 wird die Wandung des Fallschachts 20 zumindest teilweise durch optische Filter 22 gebildet. Die optischen Filter 22 bestehen vorzugsweise aus einem Material, das unempfindlich gegen elektrostatische Aufladungen ist. Für IR-Transmissionsmessungen werden vorzugsweise Rot-Grün-Filter aus FeO verwendet. Der Fallschacht 20 besitzt vorzugsweise eine charakteristische Querschnittsdimension von z. B. 2 cm. Diese kann insbesondere bei der Messstrecke 21 z. B. mit einem Einsatz 25 eingestellt werden.

Unterhalb der Messstrecke 21 ist die Sperreinrichtung 23 vorgesehen, mit der der Fallschacht 20 zumindest zeitweilig unterhalb der Messstrecke 21 verschließbar ist. Die Sperreinrichtung wird beispielsweise durch eine Klappe 23 gebildet, die um eine Achse in die Vertikale verschwenkbar ist. Die Sperreinrichtung 23 wird mit einer Stelleinrichtung 24 betätigt, die mit einer Steuereinrichtung 34 (siehe unten) verbunden ist.

Die Messeinrichtung 30 wird durch eine Lichtquelle 31 und eine Sensoreinrichtung 32 mit einem Verstärker 33 und einer Steuereinrichtung 34 gebildet. Die Lichtquelle 31 und die Sensoreinrichtung 32 bilden vorzugsweise einen Zwei-Strahl-Aufbau, wie er an sich von spektroskopischen Messverfahren bekannt ist. Einzelheiten des Zwei-Strahl-Aufbaus sind in Fig. 1 nicht dargestellt. Die Steuereinrichtung 34 ist gegebenenfalls mit einem Vergleicher 35 verbunden, der zur Betätigung der Schaufelradreinigung (siehe unten) dient. Außerdem ist eine Anzeige-, Ausgabe- und/oder Speichereinrichtung 36 vorgesehen, mit der die Messergebnisse ausgegeben werden.

Am unteren Ende des Fallschachts 20 befindet sich eine Abfuhreinrichtung, z. B. in Form einer Schublade 40.

Die Getreidefördereinrichtung 100 wird wie folgt betrieben. Vom Trichter 11 rieselt Getreide 50 auf das Schaufelrad 12. Mit dem Schaufelrad 12 wird das Getreide kammerweise in den Fallschacht 20 bewegt. Je nach den geometrischen Dimensionen des Schaufelrades 12 und des Fallschachts 20 erfolgt nach Einführen von z. B. einer, zwei oder mehreren Portionen (Kammern) in den unten geschlossenen Fallschacht 20 eine Messung der Transmission der im Bereich der Messstrecke 21 befindlichen Getreideschicht, die zum Messzeitpunkt wegen der geschlossenen Klappe 24 in der Messstrecke 21 ruht. Unmittelbar nach Aufnahme der Messwerte wird mit der Steuereinrichtung 34 die Stelleinrichtung 24 der Klappe 23 betätigt. Die vorher unbewegliche Getreideschicht fällt jetzt auf die Schublade 40 durch.

Der Ablauf aus Zufuhr von Getreide mit dem Schaufelrad 12, Messung und Freigabe der Klappe 23 kann bei geeigneter Abstimmung der Schaufelradgeschwindigkeit kontinuierlich erfolgen. Jeweils nach Leerung der letzten zu einer Messung erforderlichen Kammer des Schaufelrades 12 erfolgt unmittelbar die Messung, anschließend die Öffnung der Klappe 23, das Durchrutschen der Getreideprobe und der erneute Verschluß der Klappe 23, so dass bei Eintreffen der nächsten Getreideportion der Fallschacht 20 wieder verschlossen ist. Es kann alternativ aber auch ein diskontinuierlicher Betrieb des Schaufelrades 12 vorgesehen sein.

Die Signalauswertung erfolgt derart, dass Probenwerte oder - spektren S_{P} und Referenzwerte oder -spektren S_{R} ermittelt werden. Die Probenwerte oder -spektren werden mit einem hohen Verstärkungsfaktor des Verstärkers 33 aufgenommen. In der Steuereinrichtung 34 wird aus S_{R} und S_{P} der Differenzwert oder das Differenzspektrum S_{R} - Sp gebildet und quantitativ ausgewertet. Zur Auswertung wird beispielsweise ein an sich bekannter PLS-Algorithmus (partial least square-Algorithmus) verwendet. Unter Verwendung eines anwendungsabhängig angepassten Kalibrierungsmodell wird jedem Differenzwert oder Differenzspektrum eine vorbestimmte Getreideeigenschaft, z. B. die Feuchte oder der Proteingehalt, zugeordnet.

Wenn die Zufuhreinrichtung gestört ist, kann der jeweils ermittelte Messwert im Rahmen eines Regelkreises zur Betätigung eines Mechanismus verwendet werden, mit dem die Störung behoben wird. Wenn beispielsweise das Schaufelrad 12 klemmt oder eine Getreideprobe an einer Kammer fest anhaftet, ergeben sich Messwerte, die von erwarteten Messwerten stark abweichen. Dies wird mit dem Vergleicher 35 festgestellt. Falls der Vergleich eines Messwertes mit Vergleichsgrößen ergibt, dass kein oder zu wenig Getreide in der Messstrecke 21 liegt, so wird beispielsweise eine (nicht dargestellte) Einrichtung zur Reinigung des Schaufelrades oder eine Schütteleinrichtung betätigt.

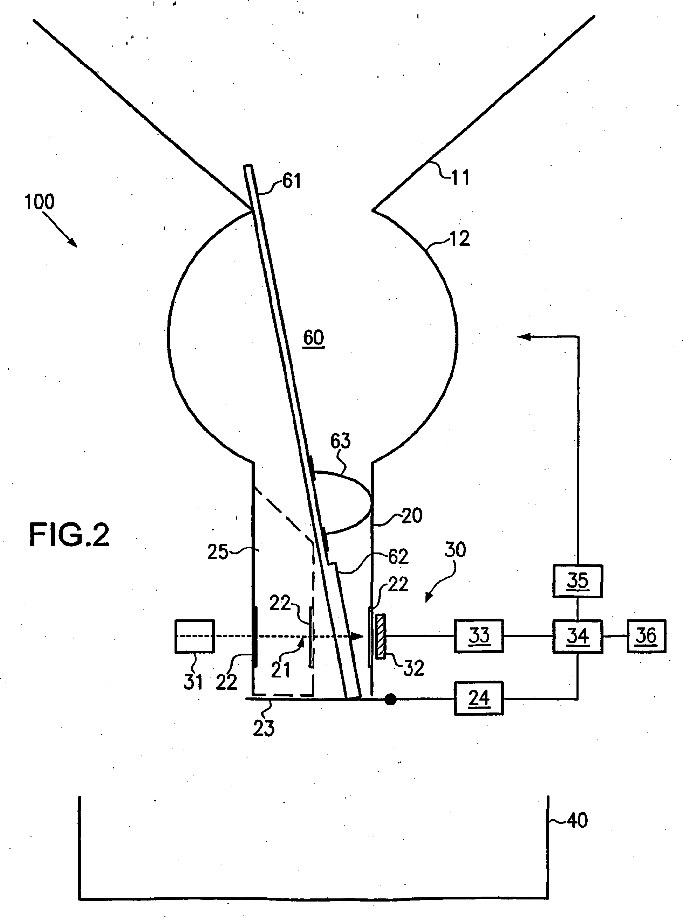

In Figur 2 (keine Ausführungsform der Erfindung) ist eine (Getreidefördereinrichtung in einem Betriebszustand dargestellt, in dem die Analyse von Getreideprodukten (z. B. Mehl) erfolgt. Hierzu wird eine Messzelle 60 verwendet, die eine Zufuhrschiene 61 und eine Schichtküvette 62 umfasst. Bei geschlossener Sperreinrichtung 23 wird die Messzelle 60 in den Fallschacht gestellt, so dass die Schichtküvette 62 von der Messstrecke 21 durchsetzt wird. Zur Fixierung der Messzelle 60 kann eine Federeinrichtung 63 vorgesehen sein. Die Federeinrichtung 63 ist je nach Bauform an der Messzelle 60 oder im Inneren des Fallschachts 20 angebracht. Im Übrigen entspricht die Getreidefördereinrichtung gemäß Figur 2 der oben beschriebenen Ausführungsform gemäß Figur 1.

Zur Messung an Getreideprodukten wird beispielsweise Mehl, Gries, Schrot oder dgl. mit der Messzelle manuell aus einem Probenvorrat aufgenommen. Die Probe gleitet die Zufuhrschiene 61 hinab bis in die Schichtküvette 62. Die mit der Probe beschickte Messzelle 60 wird nach Entnahme des Schaufelrades 12, das bspw. abschraubbar angebracht ist, in den Fallschacht eingesetzt. Anschließend erfolgt die Messung entsprechend den oben erläuterten Prinzipien.

Die Schichtküvette 62 bildet eine Schichtdicke von zum Beispiel 0.5 cm. Es kann eine flache Glasküvette oder ein zweiteiliger Aufbau aus einer Schale und einem abnehmbaren Deckel vorgesehen sein. Der zweiteilige Aufbau wird wegen der Reinigungsmöglichkeit bevorzugt.

Die Erfindung wird hier unter beispielhaftem Bezug auf eine Transmissionsmessung erläutert. In entsprechender Weise ist die Erfindung auch mit anderen spektroskopischen Messungen an einer Messstrecke eines Fallschachtes einer Getreidefördereinrichtung, z. B. mit Reflektionsmessungen, umsetzbar.

## Patentansprüche

1. Verfahren zur Messung spektroskopischer Eigenschaften von Getreidekörnern, wobei die Getreidekörner in einem Fallschacht (20) an einer Messstrecke (21) einer Erfassung von spektroskopischen Parametern des Getreides unterzogen werden, und am Fallschacht (20) eine der Messstrecke nachgeordnete Sperreinrichtung (23) derart betätigt wird, dass die Getreidekörner im Fallschacht (20) abwechselnd bewegt werden und ruhen und die Erfassung der spektroskopischen Parameter an den ruhenden Getreidekörnern erfolgt,
**dadurch gekennzeichnet, dass**
die Getreidekörner mit einem Schaufelrad das oberhalb vom Fallschacht (20) angeordnet und an diesem angebracht ist, kammerweise in vorbestimmten Portionen in den Fallschacht transportiert werden.

2. Verfahren gemäß Anspruch 1, bei dem das Schaufelrad laufend betätigt wird und eine vorbestimmte Anzahl von Portionen der Getreidekörner in den Fallschacht (20) bewegt, bis bei geschlossener Sperreinrichtung (23) eine vorbestimmte Getreideschicht an der Messstrecke (21) gebildet ist, anschließend die Erfassung der spektroskopischen Parameter erfolgt und danach die Sperreinrichtung (23) freigegeben wird.

3. Verfahren gemäß Anspruch 1, bei dem das Schaufelrad diskontinuierlich Portionen der Getreidekörner in den Fallschacht (20) bewegt.

4. Verfahren gemäß einem der vorhergehenden Ansprüche, bei dem die Sperreinrichtung (23).mit einer Stelleinrichtung (24) betätigt wird, die nach Abschlußjeweils einer Messung angesteuert wird.

5. Verfahren gemäß einem der vorhergehenden Ansprüche, bei dem das Schaufelrad in Abhängigkeit vom Messsignal einer Reinigung und/oder einem Rüttelvorgang unterzogen wird.

6. Verfahren gemäß einem der vorhergehenden Ansprüche, bei dem eine Messung der Transmission oder der optischen Dichte der in der Messstrecke (21) befindlichen Getreidekörner erfolgt.

7. Verfahren gemäß Anspruch 6, bei dem eine Differenzwert- oder -spektrenbildung erfolgt.

8. Getreidefördereinrichtung (100) mit einer Messeinrichtung (30) zur Messung spektraler Eigenschaften von Getreidekörnern, wobei ein Fallschacht (20) vorgesehen ist, an dem die Messeinrichtung (30) angeordnet ist, sowie eine am Fallschacht (20) unterhalb der Messeinrichtung (30) angeordnete Sperreinrichtung (23), mit der der Fallschacht (20) zumindest zeitweilig verschließbar ist,
**dadurch gekennzeichnet, dass**
ein Schaufelrad (12) oberhalb vom Fallschacht (20) angeordnet und an diesem angebracht ist, wobei das Schaufelrad (12) dazu eingerichtet ist, die Getreidekörner kammerweise in vorbestimmten Portionen in den Fallschacht zu bewegen.

9. Getreidefördereinrichtung gemäß Anspruch 8, bei der die Wand des Fallschachts (20) im Bereich der Messeinrichtung (30) zumindest teilweise durch optische Filter aus einem Material gebildet wird, das unempfindlich gegen elektrostatische Aufladungen ist.

10. Getreidefördereinrichtung gemäß einem der Ansprüche 8 bis 9, bei der ein Regelkreis zur Behebung von Störzuständen des Schaufelrades vorgesehen ist.

11. Getreidefordereinrichtung gemäß Anspruch 8, bei der das Schaufelrad entnehmbar ist und bei entnommenem Schaufelrad und geschlossener Sperreinrichtung eine Messzelle (60) in den Fallschacht (20) einstellbar ist.

## Claims

1. A method for measuring spectroscopic properties of cereal grains, wherein the cereal grains are subjected to a detection of spectroscopic parameters of the cereal at a measuring section (21) in a fall chute (20), and a blocking device (23) disposed at the fall chute (20) downstream of the measuring section is actuated such that the cereal grains are alternately moved and stationary in the fall chute (20) and the detection of the spectroscopic parameters takes place on the stationary cereal grains,
**characterized in that**
the cereal grains are transported in a compartmentalised manner in predetermined portions into the fall chute by means of a bucket wheel, which is disposed above the fall chute (20) and is attached thereto.

2. The method according to claim 1, in which the bucket wheel is continuously actuated and a predetermined number of portions of the cereal grains is moved into the fall chute (20) until, with the blocking device (23) closed, a predetermined cereal layer is formed at the measuring section (21), the detection of the spectroscopic parameters then takes place and afterwards the blocking device (23) is released.

3. The method according to claim 1, in which the bucket wheel discontinously moves portions of the cereal grains into the fall chute (20).

4. The method according to any one of the preceding claims, in which the blocking device (23) is actuated by means of an actuating device (24) which is activated following the completion of each measurement.

5. The method according to any one of the preceding claims, in which the bucket wheel is subjected to a cleaning and/or a vibrating process as a function of the measuring signal.

6. The method according to any one of the preceding claims, in which a measurement of the transmission or of the optical density of the cereal grains located in the measuring section (21) takes place.

7. The method according to claim 6, in which a differential value or differential spectrum formation takes place.

8. A cereal conveying device (100) with a measuring device (30) for measuring spectral properties of cereal grains,
wherein a fall chute (20) is provided, at which the measuring device (30) is disposed, as well as a blocking device (23) which is disposed at the fall chute (20) below the measuring device (30) and by means of which the fall chute (20) can be closed at least intermittently,
**characterized in that**
a bucket wheel (12) is disposed above the fall chute (20) and is attached thereto, the bucket wheel (12) being adapted to move the cereal grains in a compartmentalised manner in predetermined portions into the fall chute.

9. The cereal conveying device according to claim 8, in which the wall of the fall chute (20) in the region of the measuring device (30) is formed at least partly by optical filters of a material which is insensitive to electrostatic charges.

10. The cereal conveying device according to any one of claims 8 to 9, in which a control circuit is provided to eliminate malfunctions of the bucket wheel.

11. The cereal conveying device according to claim 8, in which the bucket wheel is removable and, with the bucket wheel removed and the blocking device closed, a measuring cell (60) can be placed in the fall chute (20).

## Revendications

1. Procédé de mesure de propriétés spectroscopiques de grains de céréales, lesdits grains de céréales étant soumis à une saisie de paramètres spectroscopiques de la céréale dans une trémie de chute (20) sur un parcours de mesure (21), et un dispositif de retenue (23) en aval du parcours de mesure étant actionné sur la trémie de chute (20) de manière que les grains de céréales soient alternativement déplacés et mis au repos dans la trémie de chute (20), et que la saisie des paramètres spectroscopiques soit réalisée sur les grains de céréales au repos,
**caractérisé**
**en ce que** les grains de céréales sont transportés en portions prédéfinies par chambre dans la trémie de chute, par une roue à palettes disposée au-dessus de la trémie de chute (20) et attachée à celle-ci.

2. Procédé selon la revendication 1, où la roue à palettes est couramment actionnée et déplace un nombre prédéfini de portions des grains de céréales dans la trémie de chute (20) jusqu'à ce que soit formée une couche de céréale prédéfinie sur le parcours de mesure (21) alors que le dispositif de retenue (23) est fermé, les paramètres spectroscopiques étant saisis ensuite, avant l'ouverture du dispositif de retenue (23).

3. Procédé selon la revendication 1, où la roue à palettes déplace de manière discontinue des portions des grains de céréales dans la trémie de chute (20).

4. Procédé selon l'une des revendications précédentes, où le dispositif de retenue (23) est actionné par un dispositif de réglage (24) qui est commandé à l'issue de chaque mesure.

5. Procédé selon l'une des revendications précédentes, où la roue à palettes est soumise à un nettoyage et/ou à un secouage en fonction du signal de mesure.

6. Procédé selon l'une des revendications précédentes, où est effectuée une mesure de la transmission ou de la densité optique des grains de céréale se trouvant sur le parcours de mesure (21).

7. Procédé selon la revendication 6, où est réalisée une formation de valeur différentielle ou de spectre différentiel.

8. Dispositif de transport de céréales (100) avec un dispositif de mesure (30) pour la mesure de propriétés spectrales de grains de céréales, où est prévue une trémie de chute (20) sur laquelle est disposé le dispositif de mesure (30), ainsi qu'un dispositif de retenue (23) disposé sur la trémie de chute (20) en dessous du dispositif de mesure (30), avec lequel la trémie de chute (20) est au moins temporairement refermable,
**caractérisé**
**en ce qu'**une roue à palettes (12) est disposée au-dessus de la trémie de chute (20) et attachée à celle-ci, ladite roue à palettes (12) étant réalisée pour déplacer les grains de céréale en portions prédéfinies par chambre dans la trémie de chute.

9. Dispositif de transport de céréales selon la revendication 8, où la paroi de la trémie de chute (20) est au niveau du dispositif de mesure (30) au moins partiellement formée par des filtres optiques réalisés dans un matériau insensible aux charges électrostatiques.

10. Dispositif de transport de céréales selon l'une des revendications 8 et 9, où un circuit de régulation est prévu pour la suppression d'états de perturbation de la roue à palettes.

11. Dispositif de transport de céréales selon la revendication 8, où la roue à palettes est amovible et où une cellule de mesure (60) est réglable dans la trémie de chute (20) lorsque la roue à palettes est retirée et le dispositif de retenue fermé.
